## Europäisches Patentamt

## European Patent Office

## Office européen des brevets

(11) Publication number: **0 072 462**
**B1**

(12)

# EUROPEAN PATENT SPECIFICATION

(45) Date of publication of patent specification: **29.10.86**

(51) Int. Cl.⁴: **A 61 K 9/08**

(21) Application number: **82106795.6**

(22) Date of filing: **27.07.82**

(54) **Pharmaceutical preparations.**

(30) Priority: **14.08.81 JP 128032/81**

(43) Date of publication of application:
**23.02.83 Bulletin 83/08**

(45) Publication of the grant of the patent:
**29.10.86 Bulletin 86/44**

(84) Designated Contracting States:
**BE CH DE FR GB IT LI NL SE**

(56) References cited:
**US-A-2 056 208**
**US-A-3 629 412**

**CHEMICAL ABSTRACTS, vol. 83, no. 4, 28th
July 1975, page 316, no. 33070q, Columbus,
Ohio, USA**

**CHEMICAL ABSTRACTS, vol. 81, no. 10, 9th
September 1974, page 342, no. 54465w,
Columbus, Ohio, USA**

(73) Proprietor: **Toko Yakuhin Industry Co., Ltd.**
**12-23, 2-chome, Honjyo-Nishi**
**Oyodo-ku Osaka (JP)**

(72) Inventor: **Kamashita, Takuzo**
**12-28, 6-chome, Tonda-cho**
**Takatsuki-shi Osaka (JP)**

(74) Representative: **Kraus, Walter, Dr. et al**
**Patentanwälte Kraus, Weisert & Partner**
**Thomas-Wimmer-Ring 15**
**D-8000 München 22 (DE)**

# 0 072 462

**Description**

The present invention relates to a pharmaceutical preparation for external use comprising a solution of a non-steroidal compound having antiphlogistic or analgesic activity and a base for external use and containing 0.5—3.0 w/w% of said non-steroidal compound. More specifically, the invention relates to preparations which are prepared by dissolving a specific non-steroidal antiphlogistic or analgesic agent in peppermint oil or a combination of peppermint oil and a salicylic acid ester and making the solution into a pharmaceutical form for external use using a base.

A great number of non-steroidal antiphlogistic or analgesic agents are known. Representative examples are ibuprofen, ketoprofen, flurbiprofen, suprofen and diclofenac. Usually the conventional pharmaceutical forms of these are orally administrable preparations and suppositories, and it is believed that any suitable form for external use is not yet known. JP—A—81616/1978 describes an indomethacin presentation as an ointment comprising a mixture thereof with a glycol, a lower alcohol and water along with a gelling agent. In US—A—3 629 412 there are disclosed pharmaceutical compositions for external use comprising indomethacin and methyl salicylate. Pharmaceutical compositions for external use comprising aspirin and methyl or ethyl salicylate are disclosed in US—A—2 056 208. In Chemical Abstracts, Volume 83, Abstract No. 33070q, 1975 (JP—A—7 535 321) and Chemical Abstracts, Vol. 81, Abstract No. 54465w (JP—A—7 430 525) there are disclosed pharmaceutical compositions for external use comprising compounds having analgesic or antiphlogistic activity and methyl salicylate and/or peppermint oil. However, the pharmaceutical compositions of these documents do not contain non-steroidal antiphlogistic or analgesic agents as mentioned above.

The above described non-steroid antiphlogistic or analgesic agents, although having an excellent antiphlogistic or analgesic effect, sometimes exhibit drastic side effects upon the digestive organs in the case of the oral administration or on the rectum in the case of the suppository, and therefore, many investigations have been made in order to eliminate such problems, but they have not yet been solved.

The present inventor has discovered as the result of intensive study that certain compounds which are non-steroid antiphlogistic or analgesic agents having a 1-carboxyethyl or carboxymethyl group in the molecule are surprisingly readily soluble in peppermint oil or a combination of peppermint oil and salicylic acid esters and such solutions exhibit pharmaceutical efficacy and that preparations for external use prepared using such solutions are extremely good as antiphlogistic or analgesic agents.

The subject matter of the present invention is a pharmaceutical preparation for external use, as defined above, which is characterized in that the non-steroidal compound [Compound (I)] is one having a 1-carboxyethyl group in the molecule and selected from ibuprofen, ketoprofen, flurbiprofen, naproxen, benoxaprofen, pranoprofen, suprofen, fenoprofen, Y9213 [2 - {4 - [2 - imidazo - (1,2-a) - pyridyl]phenyl}propionic acid], tiaprofenic acid and protizinic acid, or diclofenac, and is dissolved in peppermint oil as the only adjuvant or in a combination of adjuvants comprising peppermint oil and methyl, ethyl or monoglycol salicylate, in the ratio by weight of peppermint oil to the non-steroidal compound being 1—10 parts:1 part or the ratio by weight of peppermint oil and salicylate to the non-steroidal compound being 3—16 parts:1 part.

According to a preferred embodiment of the present invention, the ratio by weight of peppermint oil to the non-steroidal compound is 2—8 parts:1 part.

The peppermint oil used in this invention has topical vasodilating effect, anticonvulsive effect, and is used for external use in the form of an aqueous solution, poultice or plaster or used as a topical stimulant for gargling (see Oleum Menthae Japonicae, Japanese Pharmacopeia and Peppermint, British Pharmacopeia).

In this invention, the term "preparation for external use" means applying to or plastering on the skin.

The peppermint oil used in this invention is that having pharmaceutical efficacy as such and moreover being capable of dissolving the aforesaid non-steroidal antiphlogistic or analgesic agent.

The peppermint oil is that containing 1-menthol as a main ingredient, and although its composition varies to some extent depending on the plant source, any may be employed. In other words, this may be any peppermint oil included and defined in Japanese Pharmacopeia, British Pharmacopeia, West German Pharmacopeia etc.

According to this invention, Compound (I) is contained in an amount of 0.5—3.0% by weight as the active agent in the preparation for external use. Where the aforesaid adjuvant such as peppermint oil etc. is used solely as a dissolving agent, the amount just sufficient to dissolve Compound (I) may be satisfactory. The amount required is 1—10 parts by weight of pepermint oil and, if used, preferably at least 5 parts by weight of methyl salicylate or ethyl salicylate or 3—8 parts by weight of monoglycol salicylate per part by weight of Compound (I), provided that the ratio by weight of peppermint oil and salicylate to the compound (I) is 3—16 parts:1 part.

In such a case, Compound (I) may be dissolved in one of the adjuvants, and thereafter desired amounts of the other adjuvant or adjuvants are added thereto. When Compound (I) is dissolved in these adjuvants, if the amount of a single adjuvant used is too much or the amount used is not sufficient for complete dissolution, it is advisable to use two or more adjuvants to achieve dissolution, thus affording an effective adjuvant in which the features attributable to the respective adjuvants are versatile for the active ingredient.

2

**0 072 462**

Examples of the preferred form of the preparation for external use according to this invention include ointments, gels, gel creams, creams and poultices. The base for external use therefor may be chosen from known ones. By way of illustration, examples of the gel base include a dilute aqueous carboxyvinyl polymer solution, an aqueous water-soluble basic substance (e.g. sodium hydroxide) solution. Using this base, the solution of Compound (I) dissolved in peppermint oil is made into a pharmaceutical form, thereby a gel preparation is readily obtained. The carboxyvinyl polymer herein used is a hydrophilic polymer obtained by polymerization using acrylic acid as a main component, and, for example, those commercially available under the trade names such as Carbopol 934, 940, 941 from Goodrich Chemical Co., U.S.A., and Hiviswako 103, 104, 105 from Wako Junyaku Kogyo K.K., Japan may be employed.

The gel cream preparation includes those containing, in addition to the aforesaid gel base, an emulsifier (preferably a nonionic surfactant), and an oil component (e.g. liquid paraffin).

The cream base is, for example, a hydrophilic ointment (included in Japanese Pharmacopeia).

Examples of the poultice base include kaolin, glycerin, sodium acrylate, polyvinyl acetate and carboxyvinyl polymer.

Examples of the ointment base include lanolin, Vaseline (Trade Mark), beeswax and vegetable oil.

In addition to the above examples, it is possible to appropriately choose and employ bases for external use, preservatives and other additives known in the art. The conditions for preparing the preparation for external use may also be appropriately chosen and employed.

The antiphlogistic or analgesic preparation of the invention for external use can be prepared by dissolving the non-steroidal Compound (I) in the adjuvant or combination of adjuvants mentioned above in an amount at least sufficient for dissolving said Compound (I) and then forming the resultant solution into a pharmaceutical preparation using a base suitable for external use.

In this invention, it is essential to dissolve Compound (I) in the adjuvant or combination of adjuvants beforehand. Once a solution has been made, addition of a base for external use which contains appropriate amounts of water, alcohol does not cause separation of crystals of Compound (I), and thus a good preparation may be obtained. Since the peppermint oil has vasodilating effect, it is believed that Compound (I) is brought into contact with the skin while it is in solution in peppermint oil and hence the absorption thereof through the skin is promoted. In addition, refreshing feeling is offered by the peppermint oil.

The methyl salicylate, ethyl salicylate and monoglycol salicylate, if used, to promote the absorption of the active ingredient through the skin and enhance the analgesic effect when used as a counterirritant where the patient complains of pain.

This invention is more particularly described by the following Examples and tests.

Example 1

Cream preparations

The cream preparation having the below-mentioned components was prepared in the manner mentioned below.

| Components | w/w% |
| --- | --- |
| Flurbiprofen | 1.0 |
| Peppermint oil | 3.0 |
| Stearic acid | 5.0 |
| Cetanol | 5.0 |
| Liquid paraffin | 15.0 |
| White vaseline | 3.0 |
| Polyoxyethylenesorbitan monostearate | 2.0 |
| Sorbitan monostearate | 0.6 |
| Propyl p-hydroxybenzoate | 0.05 |
| Methyl p-hydroxybenzoate | 0.05 |
| Aqueous 10% triethanolamine | 3.5 |
| Sodium lauryl sulfate | 0.1 |
| Purified water | 61.7 |

Flurbiprofen was dissolved in the peppermint oil by heating to about 70—80°C. Stearic acid, cetanol, liquid paraffin, white vaseline, propyl p-hydroxybenzoate were added to the solution and heated to about 70—80°C to obtain an oil phase.

The mixture of methyl p-hydroxybenzoate, an aqueous 10% triethanolamine solution, polyoxyethylenesorbitan monostearate and sorbitan monostearate solutions, sodium lauryl sulfate and purified water was heated to 70—80°C on a water bath to obtain a solution.

The resulting solution was added in the above oil phase under sufficient stirring and cooled to give a cream preparation.

Similarly, the cream preparations having the components as shown in Table 1 were prepared, but when two or more adjuvants were used, the non-steroidal antiphlogistic or analgesic agent was dissolved in the mixture of the adjuvants prepared beforehand.

3

TABLE 1

| Component | (w/w%) | | | | | | | |
|---|---|---|---|---|---|---|---|---|
| Non-steroidal agent | | | | | | | | |
| Flurbiprofen | 0.5 | 1.0 | 1.0 | 2.0 | 3.0 | | | |
| Ibuprofen | | | | | | 1.0 | 1.0 | 2.0 |
| Ketoprofen | | | | | | | | |
| Suprofen | | | | | | | | |
| Diclofenac | | | | | | | | |
| Adjuvant | | | | | | | | |
| Peppermint oil | 1.0 | 3.0 | 2.0 | 8.0 | 6.0 | 1.0 | 6.0 | 2.0 |
| Methyl salicylate | | | | | | | | |
| Ethyl salicylate | | | 4.0 | | | | | |
| Monoglycol salicylate | | | | | 5.0 | | 10.0 | |
| Base | | | | | | | | |
| Stearic acid | 5.0 | 5.0 | 5.0 | 5.0 | 5.0 | 5.0 | 5.0 | 5.0 |
| Stearyl alcohol | | | | | | | | |
| Cetanol | 5.0 | 5.0 | 5.0 | 5.0 | 5.0 | 5.0 | 5.0 | 5.0 |
| Castor oil | | | | | | | | |
| Isopropyl myristinate | | | | | 8.0 | | | |
| Liquid paraffin | 15.0 | 15.0 | 15.0 | 15.0 | | 15.0 | 15.0 | 15.0 |
| White vaseline | 3.0 | 3.0 | 3.0 | 3.0 | 3.0 | 3.0 | 3.0 | 3.0 |
| Polyoxyethylenesorbitan monostearate | 2.0 | 2.0 | 2.0 | 2.0 | 3.3 | 2.0 | 2.0 | 2.0 |
| Sorbitan monostearate | 0.6 | 0.6 | 0.6 | 0.6 | 1.0 | 0.6 | 0.6 | 0.6 |
| Propyl p-hydroxybenzoate | 0.05 | 0.05 | 0.05 | 0.05 | 0.05 | 0.05 | 0.05 | 0.05 |
| Methyl p-hydroxybenzoate | 0.05 | 0.05 | 0.05 | 0.05 | 0.05 | 0.05 | 0.05 | 0.05 |
| Aqueous 10% triethanol-amine | 3.0 | 1.0 | 1.0 | 1.0 | | 3.0 | 1.0 | 3.0 |
| Sodium lauryl sulfate | 0.1 | 0.1 | 0.1 | 0.1 | 0.1 | 0.1 | 0.1 | 0.1 |
| Purified water | 64.7 | 64.2 | 61.2 | 58.2 | 60.5 | 64.2 | 51.2 | 62.2 |

TABLE 1 (continued)

| Component | (w/w%) | | | | | | | |
|---|---|---|---|---|---|---|---|---|
| **Non-steroidal agent** | | | | | | | | |
| Flurbiprofen | | | | | | | | |
| Ibuprofen | | | | | | | | |
| Ketoprofen | 1.0 | 1.0 | 2.0 | 2.0 | 3.0 | | | |
| Suprofen | | | | | | 0.5 | 1.0 | |
| Diclofenac | | | | | | | | 1.0 |
| **Adjuvant** | | | | | | | | |
| Peppermint oil | 1.0 | 2.0 | 4.0 | 2.0 | 7.0 | 2.0 | 8.0 | 8.0 |
| Methyl salicylate | | | | | 5.0 | | | |
| Ethyl salicylate | | | | | | | | |
| Monoglycol salicylate | | 1.0 | | | | | | |
| **Base** | | | | | | | | |
| Stearic acid | 5.0 | 5.0 | 5.0 | 5.0 | 5.0 | | | 5.0 |
| Stearyl alcohol | | | | | | 10.0 | 10.0 | |
| Cetanol | 5.0 | 5.0 | 5.0 | 5.0 | 5.0 | 5.0 | 5.0 | 5.0 |
| Castor oil | | | | | | | | 15.0 |
| Isopropyl myristinate | | | | | 8.0 | 15.0 | 15.0 | |
| Liquid paraffin | 15.0 | 15.0 | 15.0 | 15.0 | | | | |
| White vaseline | 3.0 | 3.0 | 3.0 | 3.0 | 3.0 | 5.0 | 5.0 | 3.0 |
| Polyoxyethylenesorbitan monostearate | 2.0 | 2.0 | 2.0 | 2.0 | 3.3 | 4.0 | 4.0 | 2.0 |
| Sorbitan monostearate | 0.6 | 0.6 | 0.6 | 0.6 | 1.0 | 1.2 | 1.2 | 0.5 |
| Propyl p-hydroxybenzoate | 0.05 | 0.05 | 0.05 | 0.05 | 0.05 | 0.05 | 0.05 | 0.05 |
| Methyl p-hydroxybenzoate | 0.05 | 0.05 | 0.05 | 0.05 | 0.05 | 0.05 | 0.05 | 0.05 |
| Aqueous 10% triethanol-amine | 3.0 | 1.0 | 3.0 | 3.0 | | 2.0 | 2.0 | 1.0 |
| Sodium lauryl sulfate | 0.1 | 0.1 | 0.1 | 0.1 | 0.1 | 0.2 | 0.2 | 0.1 |
| Purified water | 64.2 | 64.2 | 60.2 | 62.2 | 59.5 | 55.0 | 48.5 | 59.2 |

# 0 072 462

TABLE 1 (continued)

| Component | (w/w%) | | |
|---|---|---|---|
| **Non-steroidal agent** | | | |
| Flurbiprofen | | | |
| Ibuprofen | | | |
| Ketoprofen | | | |
| Suprofen | | | |
| Diclofenac | 1.0 | 1.0 | 1.0 |
| **Adjuvant** | | | |
| Peppermint oil | 8.0 | 10.0 | 5.0 |
| Methyl salicylate | 8.0 | | |
| Ethyl salicylate | | | |
| Monoglycol salicylate | | | 8.0 |
| **Base** | | | |
| Stearic acid | 5.0 | 5.0 | 5.0 |
| Stearyl alcohol | | | |
| Cetanol | 5.0 | 5.0 | 5.0 |
| Castor oil | | | |
| Isopropyl myristinate | | 10.0 | |
| Liquid paraffin | 15.0 | | 15.0 |
| White vaseline | 3.0 | 3.0 | 3.0 |
| Polyoxyethylenesorbitan monostearate | 2.0 | 2.0 | 2.0 |
| Sorbitan monostearate | 0.6 | 0.6 | 0.6 |
| Propyl p-hydroxybenzoate | 0.05 | 0.05 | 0.05 |
| Methyl p-hydroxybenzoate | 0.05 | 0.05 | 0.05 |
| Aqueous 10% triethanol amine | 1.0 | 1.0 | 1.0 |
| Sodium lauryl sulfate | 0.1 | 0.1 | 0.1 |
| Purified water | 51.2 | 62.2 | 54.2 |

Example 2

Gel cream preparations

The gel cream preparation having the below-mentioned components was prepared in the manner mentioned below.

| Components | w/w% |
|---|---|
| Flurbiprofen | 0.5 |
| Peppermint oil | 1.0 |
| Liquid paraffin | 20.0 |
| Lauromacrogol | 1.0 |
| Aqueous 4% carboxyvinyl-polymer (Carbopol 940) | 25.0 |
| Aqueous 2% NaOH | 20.0 |
| Aqueous 1% EDTA · 2Na | 2.0 |
| Purified water | 30.5 |

Flurbiprofen was dissolved in the peppermint oil by heating to about 70—80°C. To this solution was added liquid paraffin and lauromacrogol, which was heated to about 70—80°C on a water bath.

An aqueous 1% EDTA · 2Na (disodium ethylenediamine tetraacetate) solution and purified water were stirred into an aqueous 4% carboxyvinylpolymer solution, to which 2% sodium hydroxide was added and heated to 70—80°C on a water bath. To the resulting mixture the above flurbiprofen solution was added with good stirring. The mixture was cooled to obtain a gel cream which has a pH of 6.36 (at 25°C) and a viscosity of 34,000 mPas.

Similarly, the gel cream preparations shown in Table 2 were prepared, but when two or more adjuvants were used, they were mixed beforehand and used to dissolve the non-steroidal agent.

6

TABLE 2

| Component | (w/w%) | | | | | | | | | | | |
|---|---|---|---|---|---|---|---|---|---|---|---|---|
| **Non-steroidal agent** | | | | | | | | | | | | |
| Flurbiprofen | 0.5$^{b*}$ | 1.0 | 1.0$^{a*}$ | 2.0 | 3.0 | 3.0 | | | | | | |
| Ibuprofen | | | | | | | | | | | | |
| Ketoprofen | | | | | | | 0.5 | 1.0 | 1.0 | 1.0 | 2.0$^{c*}$ | 3.0 |
| Suprofen | | | | | | | | | | | | |
| Diclofenac | | | | | | | | | | | | |
| **Adjuvant** | | | | | | | | | | | | |
| Peppermint oil | 1.0 | 8.0 | 3.0 | 8.0 | 6.0 | 6.0 | 3.0 | 2.0 | 1.0 | 2.0 | 3.0 | 6.0 |
| Methyl salicylate | | | | | | 12.0 | 2.0 | | | 4.0 | | |
| Ethyl salicylate | | | | | | | | | | | | |
| Monoglycol salicylate | | | | | 3.0 | | | | | | | |
| **Base** | | | | | | | | | | | | |
| Liquid paraffin | | 10.0 | 10.0 | 10.0 | | | 10.0 | 10.0 | 10.0 | | 20.0 | |
| Isopropyl myristinate | 3.5 | | | | 10.0 | 10.0 | | | | 20.0 | | 10.0 |
| Castor oil | | | | | | | | | | | | |
| Lauromacrogol | 1.0 | 1.0 | 1.0 | 1.0 | 1.0 | 1.0 | 1.0 | 1.0 | 1.0 | 1.0 | 1.0 | 1.0 |
| Aqueous 4% Carbopol 940 | 25.0 | 25.0 | 25.0 | 25.0 | 25.0 | 25.0 | 25.0 | 25.0 | 25.0 | 25.0 | 25.0 | 25.0 |
| Aqueous 2% NaOH | 20.0 | 20.0 | 20.0 | 20.0 | 10.0 | 10.0 | 10.0 | 20.0 | 20.0 | 16.0 | 20.0 | 10.0 |
| Aqueous 1% EDTA·2Na | 2.0 | 2.0 | 2.0 | 2.0 | 2.0 | 2.0 | 2.0 | 2.0 | 2.0 | | 2.0 | 2.0 |
| Purified water | 47.0 | 48.0 | 38.0 | 32.0 | 40.0 | 31.0 | 46.5 | 39.0 | 40.0 | 31.0 | 27.0 | 43.0 |
| White vaseline | | | | | | | | | | 5.0 | | |

*:  [a] pH (at 28.0°C) 6.33, viscosity (mPas) 34,000
    [b] pH (at 25.5°C) 6.35, Viscosity (mPas) 31,000
    [c] pH (at 26.0°C) 6.28, Viscosity (mPas) 34,000

TABLE 2 (continued)

| Component | (w/w%) | | | | | | | | | | | | | | |
|---|---|---|---|---|---|---|---|---|---|---|---|---|---|---|---|
| **Non-steroidal agent** | | | | | | | | | | | | | | | |
| Flurbiprofen | | | | | | | | | | | | | | | |
| Ibuprofen | | | | | | | | | | | | | | | |
| Ketoprofen | 1.0 | 1.0 | 1.0 | 2.0 | 3.0 | | | | | | | | | | |
| Suprofen | | | | | | 0.5 | 1.0 | 1.0 | 1.0 | 2.0 | | | | | |
| Diclofenac | | | | | | | | | | | 0.5 | 1.0 | 1.0 | 1.0 | 2.0 |
| **Adjuvant** | | | | | | | | | | | | | | | |
| Peppermint oil | 10.0 | 8.0 | 2.0 | 3.5 | 3.0 | 3.0 | 8.0 | 2.0 | 1.0 | 8.0 | 1.0 | 2.0 | 10.0 | 8.0 | 10.0 |
| Methyl salicylate | 6.0 | | 2.0 | | | | | | | | | | | 8.0 | |
| Ethyl salicylate | | | | | | | | | | | | | | | |
| Monoglycol salicylate | | | | | 6.0 | | | | 2.0 | 7.0 | | 1.0 | | | 16.0 |
| **Base** | | | | | | | | | | | | | | | |
| Liquid paraffin | | | | 20.0 | | | | | | | | | | | |
| Isopropyl myristinate | 5.0 | 10.0 | 10.0 | | 10.0 | 20.0 | 20.0 | 20.0 | 20.0 | 20.0 | | | 10.0 | 10.0 | 5.0 |
| Castor oil | | | | | | | | | | | 20.0 | 20.0 | | | |
| Lauromacrogol | 1.0 | 1.0 | 1.0 | 1.0 | 1.0 | 1.0 | 1.0 | 1.0 | 1.0 | 1.0 | 1.5 | 1.5 | 1.0 | 1.0 | 1.0 |
| Aqueous 4% Carbopol 940 | 25.0 | 25.0 | 25.0 | 25.0 | 25.0 | 25.0 | 25.0 | 25.0 | 25.0 | 20.0 | 40.0 | 40.0 | 25.0 | 25.0 | 25.0 |
| Aqueous 2% NaOH | 10.0 | 10.0 | 10.0 | 20.0 | 10.0 | 20.0 | 20.0 | 10.0 | 10.0 | 16.0 | 15.0 | 15.0 | 10.0 | 10.0 | 10.0 |
| Aqueous 1% EDTA · 2Na | 2.0 | 2.0 | 2.0 | 2.0 | 2.0 | 2.0 | 2.0 | 2.0 | 2.0 | | 2.0 | 2.0 | 2.0 | 2.0 | 2.0 |
| Purified water | 40.0 | 43.0 | 47.0 | 26.5 | 40.0 | 28.5 | 23.0 | 39.0 | 38.0 | 21.0 | 20.0 | 17.5 | 41.0 | 35.0 | 29.0 |
| White vaseline | | | | | | | | | | 5.0 | | | | | |

0 072 462

Example 3

Gel preparation

The gel preparation having the below-mentioned components was prepared in the manner mentioned below.

| Components | w/w% |
|---|---|
| Flurbiprofen | 0.5 |
| Peppermint oil | 1.5 |
| Aqueous 4% carboxyvinyl-polymer (Carbopol 940) | 30.0 |
| Aqueous 2% NaOH | 24.0 |
| Purified water | 44.0 |

Flurbiprofen was dissolved in the peppermint oil by heating to about 70—80°C.

An aqueous 4% carboxyvinylpolymer solution was slowly added to aqueous 2% sodium hydroxide and then purified water with good stirring. The above flurbiprofen solution was added to the mixture and stirred until a homogeneous solution is obtained. The preparation has a pH of 6.54 (at 28°C) and a viscosity of 45,000 mPas.

Further the gel preparations having the components which are shown in Table 3 were prepared in the same manner as above mentioned, but when two or more adjuvants were used, the non-steroidal agent was dissolved in the mixture of the adjuvants prepared beforehand.

TABLE 3

| Component | (w/w%) | | | | | | | |
|---|---|---|---|---|---|---|---|---|
| Non-steroidal agent<br>Flurbiprofen<br>Ibuprofen<br>Ketoprofen<br>Suprofen<br>Diclofenac | 1.0 | 1.0[e*] | 1.0 | 2.0 | 3.0 | 0.5[d*] | 1.0 | 2.0 |
| Adjuvant<br>Peppermint oil<br>Methyl salicylate<br>Ethyl salicylate<br>Monoglycol salicylate | 1.0<br><br><br>2.0 | 3.0 | 2.0<br>4.0 | 6.0 | 3.0<br><br><br>6.0 | 1.0 | 2.0 | 4.0 |
| Base<br>Aqueous 4% Carbopol 940<br>Aqueous 2% NaOH<br>Purified water | 30.0<br>15.0<br>51.0 | 30.0<br>24.0<br>42.0 | 30.0<br>15.0<br>48.0 | 30.0<br>24.0<br>38.0 | 30.0<br>15.0<br>43.0 | 35.0<br>28.0<br>35.5 | 35.0<br>28.0<br>34.0 | 35.0<br>28.0<br>31.0 |

*:  [d] pH (at 28.0°C) 6.41, Viscosity (mPas) 52,000
     [f] pH (at 30.8°C) 6.75, Viscosity (mPas) 46,000.

TABLE 3 (continued)

| Component | (w/w%) | | | | | | | |
|---|---|---|---|---|---|---|---|---|
| **Non-steroidal agent** | | | | | | | | |
| Flurbiprofen | 2.0 | | | | | | | |
| Ibuprofen | | | | | | | | |
| Ketoprofen | | 0.5 | 1.0 | 1.0 | 2.0 | 3.0 | | |
| Suprofen | | | | | | | 0.5 | 1.0 |
| Diclofenac | | | | | | | | |
| **Adjuvant** | | | | | | | | |
| Peppermint oil | 3.0 | 2.0 | 2.0 | 3.0 | 6.0 | 3.0 | 4.0 | 8.0 |
| Methyl salicylate | | | 2.0 | | | | | |
| Ethyl salicylate | | | | | | | | |
| Monoglycol salicylate | 3.0 | | | | | 6.0 | | |
| **Base** | | | | | | | | |
| Aqueous 4% Carbopol 940 | 30.0 | 35.0 | 30.0 | 35.0 | 35.0 | 30.0 | 35.0 | 35.0 |
| Aqueous 2% NaOH | 24.0 | 28.0 | 15.0 | 28.0 | 28.0 | 15.0 | 28.0 | 28.0 |
| Purified water | 38.0 | 34.5 | 50.0 | 33.0 | 29.0 | 43.0 | 32.5 | 28.0 |

TABLE 3 (continued)

| Component | (w/w%) | | | |
|---|---|---|---|---|
| **Non-steroidal agent** | | | | |
| Flurbiprofen | | | | |
| Ibuprofen | | | | |
| Ketoprofen | | | | |
| Suprofen | 2.0 | | | |
| Diclofenac | | 1.0 | 1.0 | 1.0 |
| **Adjuvant** | | | | |
| Peppermint oil | 10.0 | 10.0 | 8.0 | 5.0 |
| Methyl salicylate | 10.0 | | 8.0 | |
| Ethyl salicylate | | | | |
| Monoglycol salicylate | | | | 8.0 |
| **Base** | | | | |
| Aqueous 4% Carbopol 940 | 30.0 | 30.0 | 30.0 | 30.0 |
| Aqueous 2% NaOH | 24.0 | 15.0 | 15.0 | 15.0 |
| Purified water | 24.0 | 44.0 | 38.0 | 41.0 |

Example 4

Poultice preparations

The poultice preparations having the below-mentioned components was prepared in the manner mentioned below.

| Components | w/w% |
|---|---|
| Flurbiprofen | 1.0 |
| Peppermint oil | 3.0 |
| Methyl salicylate | 0.2 |
| Kaolin finely sifted | 50.8 |
| Concentrated glycerin* | 45.0 |

* Glycerium concentratum, glycerin conc. of JP Pharmacopeia which contains glycerin of more than 98.0%.

Flurbiprofen was dissolved in the mixture of peppermint oil and methyl salicylate by heating to about 70—80°C. Concentrated glycerin which was heated was mixed with a fine kaolin dried at 110°C. To the resulting cooled mixture was added the above flurbiprofen solution under sufficient stirring, to obtain a homogeneous preparation.

Similarly the poultice preparations having the components which are shown in Table 4 were prepared.

TABLE 4

| Component | (w/w%) | | | | | | | | | | | |
|---|---|---|---|---|---|---|---|---|---|---|---|---|
| **Non-steroidal agent** | | | | | | | | | | | | |
| Flurbiprofen | 1.0 | 2.0 | 2.0 | 3.0 | | | | | | | | |
| Ibuprofen | | | | | 1.0 | | | | | | | |
| Ketoprofen | | | | | | 1.0 | 2.0 | 3.0 | | | | |
| Suprofen | | | | | | | | | 1.0 | | | |
| Diclofenac | | | | | | | | | | 1.0 | 1.0 | 3.0 |
| **Adjuvant** | | | | | | | | | | | | |
| Peppermint oil | 6.0 | 5.0 | 5.0 | 3.0 | 8.0 | 2.0 | 3.0 | 3.0 | 6.0 | 10.0 | 8.0 | 15.0 |
| Methyl salicylate | | | 5.0 | | | | | | 10.0 | | 8.0 | |
| Ethyl salicylate | 10.0 | 5.0 | | | | 3.0 | | | | | | |
| Monoglycol salicylate | | | | 6.0 | | | 3.0 | 6.0 | | | | 24.0 |
| **Base** | | | | | | | | | | | | |
| Kaolin finely sifted | 45.0 | 50.0 | 50.0 | 52.0 | 50.0 | 55.0 | 52.0 | 52.0 | 45.0 | 55.0 | 50.0 | 35.0 |
| Concentrated glycerine* | 38.0 | 38.0 | 38.0 | 36.0 | 41.0 | 39.0 | 40.0 | 36.0 | 38.0 | 34.0 | 33.0 | 23.0 |

* Glycerium concentratum, glycerin conc. of JP Pharmacopeia which contains glycerin of more than 98.0%.

**0 072 462**

Stability test of the preparations

A representative gel cream preparation of flurbiprofen (1.0%) (marked with (a) in Table 2) or gel preparation of ibuprofen (0.5%) (marked with (d) in Table 3) was subjected to a stability test. The preparation was stood for a maximum 90 days at a temperature of 40, 50 or 60°C and the non-steroidal active agent was measured using a high pressure liquid chromatograph (Shimadzu Corp. of Japan, SPD-2A, LC-3A). The results are shown in Tables 5 and 6. No change in the dosage form or decomposition of the active agent was observed in the above mentioned preparations after standing for 90 days at room temperature.

TABLE 5

The flurbiprofen (1%) gel cream preparation (a) in Example 2

| Period (day) | Temperature (°C) | Ratio of flurbiprofen detected to flurbiprofen added in the preparation (%) |
|---|---|---|
| 0 | | 100.2 |
| 30 | 40 | 100.4 |
| | 50 | 100.4 |
| | 60 | 100.3 |
| 60 | 40 | 100.0 |
| | 50 | 100.3 |
| | 60 | 99.8 |
| 90 | 40 | 100.1 |
| | 50 | 100.1 |
| | 60 | 100.0 |

TABLE 6

The ibuprofen (0.5%) gel preparation (d) in Example 4

| Period (day) | Temperature (°C) | Ratio of ibuprofen detected to ibuprofen added in the preparation (%) |
|---|---|---|
| 0 | | 99.7 |
| 30 | 40 | 99.8 |
| | 50 | 100.0 |
| | 60 | 100.0 |
| 60 | 40 | 99.9 |
| | 50 | 100.1 |
| | 60 | 99.8 |
| 90 | 40 | 100.0 |
| | 50 | 99.8 |
| | 60 | 99.7 |

Pharmacological tests

1. Inhibitory effect on UV-erythema

One group consisted of three male guinea pigs weighing 280—380 g, whose hair ($5\times5$ cm$^2$) at the right side of abdomen was sheared and shaved by electric clippers and electric razor.

The test preparation (50 mg) was applied to the resulting hairless skin in a size of 4 cm diameter, with rubbing and the applied area was covered with paper and gum tape. After standing for an hour, the covering was removed and the applied test preparation was also wiped off with warm water. Then, the three parts (each diameter of 7 mm) in the applied skin were irradiated by ultraviolet rays (an artificial sunlight of 600W provided by Amako Ika Ltd. of Japan) for 90 seconds. Two hours after the irradiation, the extent of UV-erythema was evaluated, by reference to a standard having no irradiation.

12

0: no change
1: slightly reddish
2: apparent erythema with a clear border
3: strong erythema with a clear border.

The results are shown in Table 7.

2. Inhibitory effect on carrageenin edema
One group consisted of three male rats weighing 195—240 g. The test preparation was well applied to the skin of sole of left hind leg, which was covered. After standing for an hour, 0.1 ml of 1% carrageenin physiologic saline solution was subcutaneously injected into the sole. Four hours after the injection, the volume of edema was measured to calculate inhibition ratio.
The results are shown in Table 7.

13

TABLE 7

| Preparation | Concentration of active ingredient (%) | UV-Erythema inhibitory effect (50 mg application) | | Carrageenin edema inhibitory effect (50 mg application) | |
|---|---|---|---|---|---|
| | | Mean score ±S.E. | Inhibition ratio (%) | Mean volume of edema ±S.E. (ml) | Inhibition ratio (%) |
| No application | — | 1.7±0.37 | — | 0.55±0.04 | — |
| Reference gel preparation | 1.0 | 0.4±0.18** | 76.5 | 0.45±0.01 | 18.2 |
| Flurbiprofen gel preparation (e in Table 3) | base alone 1.0 | 1.3±0.17 0.6±0.18** | 23.5 64.7 | 0.65±0.05 0.31±0.05** | 18.2 43.6 |
| Flurbiprofen gel cream preparation (a in Table 2) | base alone 1.0 | 1.1±0.18 0.9±0.20** | 17.6 47.1 | 0.51±0.05 0.25±0.08** | 7.3 54.5 |

Note 1)**: means that the preparation has significant difference from "no application" at P<0.01 by t-test.
2) Reference gel cream preparation:

| | |
|---|---|
| Hiviswako 104 (Wako Junyaku K.K., Japan) | 1.0 g |
| Indomethacin | 1.0 g |
| Propylene glycol | 10.0 g |
| Ethanol | 40.0 g |
| Diisopropanolamine | 1.1 g |
| Purified water | 47.9 g |

**0 072 462**

Conclusions

With respect to UV-erythema, both of the preparations of the invention and the reference gel preparation showed significantly high inhibitory effect, compared with the no application case.

On the other hand, the preparations of the invention showed a significantly high inhibitory effect on carrageenin edema in comparison with the control (no application). However the reference gel preparation did not show any significant difference from the control.

It is found that the preparations of the invention are well absorbed into the deep subcutaneous part to exert their own pharmacological activities, in addition to curing an inflammation at the skin surface.

**Claims**

1. A pharmaceutical preparation for external use comprising a solution of a non-steroidal compound having antiphlogistic or analgesic activity and a base for external use and containing 0.5—3.0 w/w% of said non-steroidal compound, characterized in that the non-steroidal compound is one having a 1-carboxyethyl group in the molecule and selected from ibuprofen, ketoprofen, flurbiprofen, naproxen, benoxaprofen, pranoprofen, suprofen, fenoprofen, Y-9213 [2 - {4 - [2 - imidazo - (1,2-a) - pyridyl]phenyl}propionic acid], tiaprofenic acid and protizinic acid, or is diclofenac, and is dissolved in peppermint oil as the only adjuvant or in a combination of adjuvants comprising peppermint oil and methyl, ethyl or monoglycol salicylate, in the ratio by weight of peppermint oil to the non-steroidal compound of 1—10 parts:1 part or in the ratio by weight of peppermint oil and salicylate to the non-steroidal compound of 3—16 parts:1 part.

2. A pharmaceutical preparation according to claim 1 wherein the ratio by weight of peppermint oil to the non-steroidal compound is 2—8 parts:1 part.

3. A pharmaceutical preparation according to claim 1 which is in the form of a gel, gel cream, cream or poultice.

**Patentansprüche**

1. Pharmazeutische Zubereitung für den äußeren Gebrauch, die eine Lösung einer steroidfreien Verbindung mit antiphlogistischer oder analgetischer Aktivität und eine Base für den äußeren Gebrauch umfaßt und 0,5 bis 3,0% (Gewicht/Gewicht) der genannten steroidfreien Verbindung enthält, dadurch gekennzeichnet, daß die steroidfreie Verbindung eine solche ist, die eine 1-Carboxyethylgruppe im Molekül besitzt und aus Ibuprofen, Ketoprofen, Flurbiprofen, Naproxen, Benoxaprofen, Pranoprofen, Suprofen, Fenoprofen, Y-9213 [2 - {4 - [2 - Imidazo - (1,2-a) - pyridyl]phenyl}propionsäure], Thiaprofensäure und Protizinsäure ausgewählt ist, oder eine solche ist, die eine Essigsäuregruppe im Molekül aufweist, nämlich Diclofenac ist und in Pfefferminzöl als einzigem Adjuvans oder in einer Kombination von Adjuvantien, umfassend Pfefferminzöl und Methyl, Ethyl oder Monoglykolsalicylat, aufgelöst ist, wobei das Gewichtsverhältnis von Pfefferminzöl zu der steroidfreien Verbinudng 1 bis 10 Teile:1 Teil beträgt oder wobei das Gewichtsverhältnis von Pfefferminzöl und Salicylat zu der steroidfreien Verbindung 3 bis 16 Teile:1 Teil beträgt.

2. Pharmazeutische Zubereitung nach Anspruch 1, dadurch gekennzeichnet, daß das Gewichtsverhältnis von Pfefferminzöl zu steroidfreier Verbindung 2 bis 8 Teile:1 Teil beträgt.

3. Pharmazeutische Zubereitung nach Anspruch 1, dadurch gekennzeichnet, daß sie in Form eines Gels, einer Gelcreme, einer Creme oder einer Packung vorliegt.

**Revendications**

1. Préparation pharmaceutique à usage externe comprenant une solution d'un composé non-stéroïde ayant une activité antiphlogistique ou analgésique et une base à usage externe et contenant 0,5 à 3,0% en p/p de ce composé non-stéroïde, caractérisée en ce que le composé non-stéroïde est un composé ayant un groupe 1-carboxyéthyle dans la molécule et est choisi parmi l'ibuprofène, le cétoprofène, le flurbiprofène, le naproxène, le benoxaprofène, le pranoprofène, le suprofène, le fénoprofène, Y-9213 [acide 2 - {4 - [2 - imidazo - (1,2-a) - pyridyl]phényl}propionique], l'acide thiaprofénique et l'acide protizinique ou le diclofénac et qu'il est dissous dans de l'essence de menthe comme unique adjuvant ou dans une combinaison d'adjuvants comprenant de l'essence de menthe et du salicylate de méthyle, d'éthyle ou de monoglycol, selon un rapport pondéral d'essence de menthe au composé non-stéroïde de 1—10 parties:1 partie ou selon un rapport pondéral d'essence de menthe et salicylate au composé non-stéroïde de 3—16 parties:1 partie.

2. Préparation pharmaceutique selon la revendication 1, dans laquelle le rapport pondéral d'essence de menthe au composé non-stéroïde est de 2—8 parties:1 partie.

3. Préparation pharmaceutique selon la revendication 1, qui est sous forme d'un gel, d'un gel-crème, d'une crème ou d'un cataplasme.